(19) Europäisches Patentamt
European Patent Office
Office européen des brevets

(11) Veröffentlichungsnummer: **0 421 199 A2**

(12) # EUROPÄISCHE PATENTANMELDUNG

(21) Anmeldenummer: **90118104.0**

(22) Anmeldetag: **20.09.90**

(51) Int. Cl.[5]: **C07D 233/60**, A61K 31/415

(30) Priorität: **22.09.89 DE 3931554**

(43) Veröffentlichungstag der Anmeldung:
**10.04.91 Patentblatt 91/15**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Kampe, Klaus-Dieter, Dr.**
**Am Rehsteig 1**
**W-6232 Bad Soden am Taunus(DE)**
Erfinder: **Gerhards, Hermann Josef, Dr.**
**Wacholderweg 4**
**W-6238 Hofheim am Taunus(DE)**
Erfinder: **Stechl, Jens, Dr.**
**Loreleistrasse 7**
**W-6230 Frankfurt am Main 80(DE)**
Erfinder: **Usinger, Patricia, Dr.**
**Am Dornbusch 20**
**W-6239 Eppstein(DE)**

(54) **( + )-(2-Hydroxy-2-adamantyl)-1-imidazolyl-3-tolyl-methan und dessen Salze, Verfahren zu dessen Herstellung, diese Verbindung enthaltende Arzneimittel und dessen Verwendung.**

(57) Die Erfindung betrifft das rechtsdrehende, das ( + )-Enantiomere des (2-Hydroxy-2-adamantyl)-1-imidazolyl-3-tolyl-methans sowie dessen physiologisch verträglichen Säureadditionssalze.

Die genannten Verbindungen werden als Psychopharmaka, insbesondere als Antidepressiva, verwendet.

Weiterhin werden pharmazeutische Zubereitungen beschrieben, die die genannten Verbindungen als Wirkstoffe enthalten. Die pharmazeutischen Zubereitungen können oral, parenteral, intraperitoneal und/oder rectal appliziert werden.

EP 0 421 199 A2

# ( + )-(2-HYDROXY-2-ADAMANTYL)-1-IMIDAZOLYL-3-TOLYL-METHAN UND DESSEN SALZE, VERFAHREN ZU DESSEN HERSTELLUNG, DIESE VERBINDUNG ENTHALTENDE ARZNEIMITTEL UND DESSEN VERWENDUNG

Die Erfindung betrifft das rechtsdrehende, das ( + )-Enantiomere des (2-Hydroxy-2-adamantyl)-1-imidazolyl-3-tolyl-methans einschließlich dessen Salze, Verfahren zu dessen Herstellung, diese Verbindung enthaltende Arzneimittel und dessen Verwendung als Arzneimittel.

Insbesondere betrifft die Erfindung die physiologisch verträglichen Salze des( + )-Enantiomeren des (2-Hydroxy-2-adamantyl)-1-imidazolyl-3-tolyl-methans.

Es ist bereits beschrieben worden (Deutsche Patentanmeldungen P 36 28 545.5 und P 37 07 151.3), daß substituierte Arylmethylazole und deren Salze, die aus der Umsetzung von 1-(Arylmethyl)-azolen mit Ketonen und Aldehyden hervorgehen, aufgrund ihrer psychopharmakologischen, insbesondere ihrer antidepressiven Wirkung zur Behandlung von depressiven Zuständen geeignet sind.

Es wurde nun überraschenderweise gefunden, daß das rechtsdrehende, das ( + )-Enantiomere von (2-Hydroxy-2-adamantyl)-1-imidazolyl-3-tolyl-methan der Formel I sowie dessen physiologisch verträgliche Säureadditionsalze, insbesondere das Hydrochlorid und das (2S,3S)-Hydrogentartrat, ganz besonders ausgeprägte Wirkungen in für antidepressive Wirkung beim Menschen prädiktiven Tiermodellen zeigen.

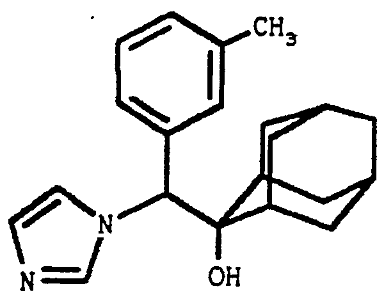

I

(2-Hydroxy-2-adamantyl)-1-imidazolyl-3-tolyl-methan in Form des Racemats, wie es bei der chemischen Synthese anfällt, ist bereits in der Deutschen Patentanmeldung P 36 28 545.5 als, in prädiktiven Tiermodellen, antidepressiv wirksam genannt worden.

Bei der pharmakologischen Untersuchung des ( + )-Enantiomeren der Verbindung der Formel I wurden unerwartet starke Wirkungen dieser Substanz in Tiermodellen, die als prädiktiv für antidepressive Wirkung beim Menschen gelten, gefunden. Der andere Antipode das linksdrehende, das (-)-Enantiomere der Verbindung I ist dagegen pharmakologisch unwirksam.

Es ist außerordentlich überraschend, daß gerade das ( + )-Enantiomer der Formel I ebenfalls eine gute antidepressive Wirkung zeigt, weil andere ( + )-Enantiomere aus der gleichen Verbindungsklasse, beispielsweise das ( + )-(2-Hydroxy-2-adamantyl)-1-imidazolyl-(3-chlorphenyl)-methan, im Vergleich zu den entsprechenden Racematen geringere Wirkung zeigen (siehe auch Vergleichsbeispiele 1 und 2). Die gute Wirksamkeit des ( + )-Enantiomeren der Formel I war also keineswegs vorhersehbar, im Gegenteil, der Fachmann mußte aus der geringeren Wirkung des ( + )-(2-Hydroxy-2-adamantyl)-1-imidazolyl-(3-chlorphenyl)-methans schließen, daß auch andere ( + )-Enantiomere aus dieser Stoffklasse nicht besser wirken als die Racemate.

Die pharmakologischen Prüfungen wurden mit bekannten Modellen, die als prädiktiv für antidepressive Wirkung beim Menschen gelten, durchgeführt. Das sind beispielsweise: die Beeinflussung der Tetrabenazin-Ptosis bei der Maus und die Potenzierung der Yohimbin-Toxizität bei Mäusen. Als weiteres Beurteilungskriterium wurde beispielsweise die Prüfung der Auslösung von Stereotypien an Ratten herangezogen.

Die Verbindung der Formel I als reines ( + )-Enantiomer wurde bei der pharmakologischen Prüfung mit Hilfe vorstehend angeführter Tiermodelle mit anderen, in diesen Modellen gut wirksamen Verbindungen aus der Klasse der 1-(1-Phenyl-2-hydroxy-ethyl)-imidazole, wie sie in den Deutschen Patentanmeldungen P 36 28 545.5 und P 37 07 151.3 beschrieben sind, eingeschlossen die racemische Verbindung I, verglichen.

Wie aus den nachstehenden Tabellen hervorgeht, ist das rechtsdrehende, das ( + )-Enantiomere der Verbindung I in allen Testmodellen gegenüber den anderen genannten Verbindungen der Stoffklasse, darunter auch ein reines ( + )-Enantiomer, überlegen.

Die Erfindung betrifft auch ein Verfahren zur Herstellung des rechtsdrehenden Enantiomeren des (2-Hydroxy-2-adamantyl)-1-imidazolyl-3-tolyl-methans der Formel I, das dadurch gekennzeichnet ist, daß man das Racemat der Verbindung I mit einem Äquivalent (2S,3S)-(-)-Weinsäure in einem geeigneten Lösungsmittel oder Lösungsmittelgemisch in das Hydrogentartrat überführt und das gebildete diastereomere Hydrogentartrat aus einem geeigneten Lösungsmittel oder Lösungsmittelgemisch fraktioniert kristallisieren läßt und anschließend das auskristallisierte rechtsdrehende (2-Hydroxy-2-adamantyl)-1-imidazolyl-3-tolyl-methan-(2S,3S)-hydrogentartrat ein-oder mehrmals aus einem geeigneten Lösungsmittel oder Lösungsmittelgemisch umkristallisiert bis die spezifische Drehung konstant bleibt.

Als Lösungsmittel sind im Prinzip alle herkömmlichen organischen Lösungsmittel oder Gemische solcher Lösungsmittel, gegebenenfalls auch solche, die, sofern mit Wasser mischbar, einen Wasseranteil von bis zu 15 Vol.-% enthalten, verwendbar. Vorteilhaft werden für die Racematspaltung der Verbindung I Tetrahydrofuran, 1,2-Dimethoxyethan, Dioxan, Acetonitril, Aceton, Methylethylketon, Methylisobutylketon oder Methyl- oder Ethylacetat unter Zumischen von bis zu 30 Vol.-% Methanol oder Ethanol verwendet. Besonders geeignet als Lösungsmittel sind Mischungen von Aceton und/oder Acetonitril mit einem Gehalt von 4 - 20 Vol.-% Methanol. Das erfindungsgemäße Verfahren kann innerhalb eines sehr breiten Temperaturbereichs ausgeführt werden. Zweckmäßig wird die Bildung des Hydrogentartrats des Racemats der Verbindung I bei einer Temperatur zwischen Zimmertemperatur und dem Siedepunkt des Lösungsmittels vorgenommen. Zur Auskristallisation des gebildeten Hydrogentartrats wird, vorteilhaft langsam, d.h. innerhalb von mehreren Stunden, bis zu einer Temperatur, die einige Grad Celsius oberhalb des Erstarrungspunktes der Lösung liegt, vorteilhaft bis zu einer Temperatur zwischen +5 °C und +30 °C, abgekühlt.

Aus dem erfindungsgemäß erhaltenen, reinen (+)-(2-Hydroxy-2-adamantyl)-1-imidazolyl-3-tolyl-methan-(2S,3S)-hydrogentartrat kann die freie Base, das (+)-(2-Hydroxy-2-adamantyl)-1-imidazolyl-3-tolyl-methan, nach im Prinzip bekannten Methoden, beispielsweise durch Zusatz einer starken Base wie eines Alkali- oder Erdalkalihydroxids oder einer starken organischen Base, wie einem Trialkylammoniumhydroxid oder einem Trialkylamin, beispielsweise Triethylamin, zweckmäßig in Gegenwart von Wasser und vorteilhaft in Gegenwart eines mit Wasser nicht mischbaren organischen Lösungmittels, wie beispielsweise Dichlormethan, in dem sich die Verbindung I zumindest anteilweise löst, freigesetzt werden. Die Freisetzung der Verbindung I aus ihren Säureadditionssalzen, wie beispielsweise dem (2S,3S)-Hydrogentartrat oder dem Hydrochlorid kann auch mit Hilfe eines basischen, vorteilhaft stark basischen, Ionenaustauschers erfolgen.

Das (+)-(2-Hydroxy-2-adamantyl)-1-imidazolyl-3-tolyl-methan, die Verbindung I, kann durch Zusatz einer äquivalenten Menge einer physiologisch verträglichen Säure, zweckmäßig unter Mitverwendung eins Lösungs- bzw. flüssigen Verdünnungsmittels, in Säureadditionssalze übergeführt werden.

Zur Bildung von Säureadditionssalzen kommen alle Säuren, die physiologisch verträgliche Salze bilden, in Frage. Dazu gehören sowohl anorganische Säuren als auch mono-, bi- und trifunktionelle organische Säuren, wie beispieslweise Chlor-, Brom- oder Jodwasserstoff-, Schwefel-, Phosphor-, Salpeter-, Benzolsulfon-, Toluolsulfon-, Sulfamin-, Methylschwefel-, Essig-, Propion-, Öl-, Palmitin-, Stearin-, Malon-, Malein-, Bernstein-, Glutar-, Äpfel-, Wein-, Zitronen-, Fumar-, Milch-, Glykol-, Brenztrauben-, Benzoe-, Toluyl-, Glutamin-, Furancarbon-, Salicyl- oder Mandelsäure. Bevorzugt werden Salze mit physiologisch verträglichen anorganischen Säuren, stark bis mittelstark sauren Derivaten solcher Säuren oder mit Bernsteinsäure, L(+)- oder D(-)-Weinsäure, Äpfelsäure, Fumarsäure, (S)-(+)-oder (R)-(-)-Mandelsäure.

Wie aus den Ergebnissen der pharmakologischen Prüfung hervorgeht, erwies sich das (+)-Enantiomere der Verbindung I, das in Form des (2S,3S)-Hydrogentartrats geprüft wurde, anderen ausgeprägt antidepressiv wirkenden Verbindungen der gleichen Stoffklasse und Nomifensin gegenüber als überlegen.

(+)-(2-Hydroxy-2-adamantyl)-1-imidazolyl-3-tolyl-methan, die erfindungsgemäße Verbindung und ihre Säureadditionssalze zeichnen sich, abgeleitet aus ihren Effekten in prädiktiven Tiermodellen, als besonders antidepressiv wirkende Substanzen aus, die somit wertvolle Psychopharmaka darstellen und zur Behandlung von depressiven Zuständen als Arzneimittel verwendet werden können. Die Höhe der verabreichten Dosis ist abhängig von der Art der gewünschten Behandlung, von der Verabreichungweise, vom Zustand, vom Typ und von dem Gewicht des behandelten Individuums. Bei oraler Dosierung werden befriedigende Ergebnisse mit Dosen ab 0,1 mg, vorzugsweise ab 0,4 mg, bis zu 60 mg, vorzugsweise bis zu 15 mg, einer Verbindung der Formel I pro kg Körpergewicht erreicht.

Die erfindungsgemäßen Verbindungen antagonisieren mit einer $ED_{50}$ von 0,8 - 1,9 mg/kg oral appliziert, die Tetrabenazin-Ptosis an der Maus. Sechs männl. Tieren wird die entsprechende Menge eines Homogenats der jeweiligen Substanz in wäßriger Carboxymethylcellulose oder Methyl-hydroxyethylcellulose 30 Min. vor der Behandlung mit Tetrabenazin (40 mg/kg intraperitoneal) mit Schlundsonde gefüttert. Die Tetrabenazin-Ptosis wird durch diese Vorbehandlung antagonisiert.

Die Verbindung I als (+)-Enantiomer und dessen Säureadditionssalze potenzieren die Toxizität von Yohimbin an Mäusen mit einer $ED_{50}$ von 2,0 - 5,0 mg/kg, oral appliziert.

Ein wesentlicher Befund ist, daß die erfindungsgemäße Verbindung I und ihre Salze an Ratten und

Mäusen nach Dosierung in einem therapeutisch relevanten Bereich keine Stereotypien verursachen.

Die antidepressive Wirkung und die Brauchbarkeit zur Behandlung von depressiven Zuständen wurde weiterhin anhand der Hemmung der Wiederaufnahme von Noradrenalin an Mäusehirn-Synaptosomen-Präparationen demonstriert.

Das (+)-Enantiomer der Verbindung I sowie dessen Säureadditionssalze sind besonders wertvoll durch eine von den bisher bekannten Antidepressiva abweichende Struktur. Bekannten Handelsprodukten ist es in seiner Wirkung bei geringerer Toxizität gleichwertig oder überlegen.

Zur vorliegenden Erfindung gehören pharmazeutische Zubereitungen, die neben nicht toxischen, inerten pharmazeutisch geeigneten Trägerstoffen, den erfindungsgemäßen Wirkstoff enthalten oder die aus dem erfindungsgemäßen Wirkstoff bestehen sowie Verfahren zur Herstellung dieser Zubereitungen.

Unter nicht toxischen, inerten, pharmazeutisch geeigneten Trägerstoffen sind feste, halbfeste oder flüssige Verdünnungsmittel, Füllstoffe und Formulierungshilfsmittel jeder Art zu verstehen.

Als geeignete Anwendungsformen der erfindungsgemäßen Verbindungen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen in Betracht.

Die therapeutisch wirksamen Verbindungen sollen in den oben aufgeführten pharmazeutischen Zubereitungen zweckmäßig in einer Konzentration von etwa 0,01, vorzugsweise von 0,10 bis 99,0, vorzugsweise bis 50,0 Gewichtsprozenten der Gesamtmischung vorhanden sein.

Die oben aufgeführten pharmazeutischen Zubereitungen können außer den erfindungemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Der Wirkstoff oder die pharmazeutischen Zubereitungen können oral, parenteral, intraperitoneal und/oder rectal appliziert werden.

Die als Antidepressivum verwendbare Verbindung der vorliegenden Erfindung und ihre Salze können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Gelatinekapseln, welche den Wirkstoff zusammen mit Verdünnungsmitteln, z.B. Lactose, Dextrose, Rohrzucker, Mannitol, Sorbitol, Cellulose und/oder Glycin und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumaluminiumsilicat, Stärke, Gelatine, Traganat, Methylcellulose, Natriumcarboxymethylcellulose und/oder Polyvinylpyrrolidon und, falls benötigt, Farbstoffe, Geschmacksstoffe und Süßmittel. Injizierbare Lösungen sind vorzugsweise isotonische wäßrige Lösungen oder Suspensionen, die sterilisiert sein können und Hilfsstoffe wie Konservier-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die, wenn erwünscht pharmakologisch wertvolle Stoffe enthalten können, werden z.B. mittels konventioneller Misch-,Granulier- und Dragierverfahren, hergestellt und enthalten 0,1 % bis etwa 75 % bevorzugt etwa 1 % bis etwa 50 % des Wirkstoffs.

Die orale Anwendung erfolgt in pharmazeutisch üblichen Zubereitungen, beispielsweise in Form von Tabletten, Dragees oder Kapseln, die z.B. pro Tagesdosis 5, vorzugsweise 15, bis 300 mg, vorzugsweise bis 150 mg, des Wirkstoffes in Mischung mit einem gebräuchlichen Trägerstoff und/oder Konstituents enthalten, wobei Einzeldosen von 5 bis 50 mg, vorzugsweise ein- bis dreimal täglich, gegeben werden können.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die folgenden Beispiele erläutern die Herstellung der Verbindungen.

**Beispiel 1**

(+)-(2-Hydroxy-2-adamantyl)-1-imidazolyl-3-tolyl-methan-(2S,3S)-hydrogentartrat:

20,44 g (63,4 mmol) (2-Hydroxy-2-adamantyl)-1-imidazolyl-3-tolyl-methan (Herstellung gemäß Deutscher Patentanmeldung P 36 28 545.5, Beispiel 106) und 9,52 g (63,4 mmol) (2S,3S)-(-)-Weinsäure wurden in 125 ml Aceton bei Siedetemperatur gelöst. Beim langsamen Abkühlen der Lösung auf Zimmertemperatur erfolgte Kristallisation. Nach einem Tag (24 h) wurde das Kristallisat abgesaugt und mit Aceton ausgewaschen und 15 Std. bei 98 °C/4-7 mbar getrocknet. Man erhielt 10,3 g (2-Hydroxy-2-adamantyl)-1-imidazolyl-3-tolyl-methan-(2S,3S)-hydrogentartrat ($[\alpha]_D^{22}$ : (+) 6,09 ° (C = 1,0; $CH_3OH$)). Dieses Produkt löste man in einer siedenden Mischung von 130 ml Aceton und 13 ml Methanol und ließ bei langsamer Abkühlung auf Zimmertemperatur auskristallisieren. Nach einem Tag (24 h) wurde wie vorstehend beschrieben das Kristallisat abgesaugt, ausgewaschen und getrocknet. Man erhielt 6,32 g ($[\alpha]_D^{22}$ : (+) 6,57 ° (C = 1,0; $CH_3OH$)) Produkt. Diese 6,32 g wurden in analoger Weise aus 80 ml Aceton und 8 ml Methanol umkristallisiert, wobei 4,68 g Produkt ($[\alpha]_D^{22}$ : (+) 6,90 ° (C = 1,0; $CH_3OH$)) erhalten wurden. Eine weitere Umkristallisation aus 95 ml Aceton und 6 ml Methanol lieferte 3,48 g (≙ 23,2 % d. Th.) reines (+)-(2-Hydroxy-2-adamantyl)-1-imidazolyl-3-tolylmethan-(2S,3S)-hydrogentartrat, $[\alpha]_D^{22}$ : (+) 6,93 ° (C = 1,0 ; $CH_3OH$); Fp. 115 - 117 °C.

Die Mutterlaugen der letzten und vorletzten Umkristallisation wurden zusammengefaßt, im Vakuum eingedampft und der verbliebene Rückstand (2,60 g) wurde in analoger Weise wie vorstehend beschrieben aus 30 ml Aceton und 3 ml Methanol umkristallisiert. Man erhielt 1,9 g Produkt, das in analoger Weise noch zweimal aus entsprechenden Aceton/Methanol-Mischungen umkristallisiert wurde. Danach wurden weitere 1,20 g (≙ 8,0 % d. Th.) reines (+)-(2-Hydroxy-2-adamantyl)-1-imidazolyl-3-tolyl-methan-(2S,3S)-hydrogentartrat, $[\alpha]_D^{22}$ : (+) 6,92 ° (C = 1,0; $CH_3OH$); Fp. 115 - 117 °C, erhalten. Insgesamt sind auf diese Weise 4,68 g (≙ 31,2 % d. Th.) des reinen (2S,3S)-Hydrogentartrats vom (+)-Enantiomeren der Verbindung I erhalten worden.

| $C_{25}H_{32}N_2O_7$ (472,55) | | | | |
|---|---|---|---|---|
| ber.: | C 63,54 | H 6,83 | N 5,93 | O 23,70 % |
| gef.: | C 63,2 | H 6,4 | N 5,9 | O 23,5 % |

**Beispiel 2**

(+)-(2-Hydroxy-2-adamantyl)-1-imidazolyl-3-tolyl-methan:

Eine Mischung aus 7,09 g (15 mmol) (+)-(2-Hydroxy-2-adamantyl)-1-imidazolyl-3-tolyl-methan-(2S,3S)-hydrogentartrat (hergestellt gemäß Beispiel 1), 150 ml $CH_2Cl_2$, 16,5 ml 2n Natronlauge und 20 ml Wasser wurde solang bei Zimmertemperatur geschüttelt bis zwei klare Phasen entstanden waren. Nach Trennung der Phasen wurde die wäßrige zweimal mit $CH_2Cl_2$ extrahiert. Die vereinigten $CH_2Cl_2$-Phasen wurde einmal mit Wasser ausgewaschen, getrocknet, filtriert und im Vakuum eingedampft. Der amorphe, schaumartige Rückstand wurde mit 100 ml Ether 10 Min. zum Sieden erhitzt, wobei ein kristalliner Feststoff entstand. Dieser wurde abgesaugt, mit Ether ausgewaschen und getrocknet. Man erhielt 4,35 g (90 % d. Th.) reines (+)-(2-Hydroxy-2-adamantyl)-1-imidazolyl-3-tolyl-methan, $[\alpha]_D^{22}$ : (+) 23,90 ° (C = 1,0; $CH_3OH$); Fp. 199 - 200 °C.

| $C_{21}H_{26}N_2O$ (322,46) | | | |
|---|---|---|---|
| ber.: | C 78,22 | H 8,13 | N 8,69 % |
| gef.: | C 77,6 | H 8,1 | N 8,6 % |

**Beispiel 3**

(+)-(2-Hydroxy-2-adamantyl)-1-imidazolyl-3-tolyl-methan-hydrochlorid:

6,45 g (20 mmol) (+)-(2-Hydroxy-2-adamantyl)-1-imidazolyl-3-tolyl-methan (hergestellt gemäß Beispiel 2) wurden in 92 ml Ethylacetat und 5 ml Methanol unter Erwärmen gelöst, auf Zimmertemperatur abgekühlt und tropfenweise mit 3,8 ml einer 5,9 molaren Lösung von HCl in Ether versetzt. Nach einigen Minuten fiel kristalliner Niederschlag aus. Man rührte 1 Std. unter Eisbad-Kühlung nach, saugte das Kristallisat ab und wusch mit kaltem Ethylacetat und Ether nach. Nach dem Trocknen der Substanz wurden 6,32 g (≙ 88 % d. Th.) reines (+)-(2-Hydroxy-2-adamantyl)-1-imidazolyl-3-tolyl-methan-hydrochlorid erhalten, $[\alpha]_D^{22}$ :(+) 15,31 ° (C = 0,993, $CH_3OH$);
Fp. 194 - 195 °C.

| $C_{21}H_{27}ClN_2O$ (358,92) | | | | |
|---|---|---|---|---|
| ber.: | C70,27 | H 7,58 | Cl 9,88 | N 7,81 |
| gef.: | C 69,6 | H 7,7 | Cl 9,4 | N 7,6 |

## Pharmakologische Prüfung

1. Prüfung auf Beeinflussung der Tetrabenazin-Ptosis (TBZ Methode)

Versuch an Mäusen (NMRI) männlich, Gewicht 18-22 g:

Männliche Mäuse (SPF-71, KF:NMRI); Gewicht 18-22 g, wurden randomisiert Behandlungsgruppen á 6 Tieren zugeteilt. Die Prüfsubstanzen wurden in 1 %iger Tylose/Wasser suspendiert und oral in 10 ml/kg Körpergewicht verabreicht. Eine Kontrollgruppe erhielt das Lösungs- bzw. Suspendiermittel (1 % Tylose).

Die verwendete Tetrabenzazin-Lösung wurde vom Methan-Sulfonat (≙ 76,8 % Base) hergestellt und eine Menge entsprechend 40 mg/kg Tetrabenazin-Base intraperitoneal 30 Min. nach den Prüfsubstanzen appliziert. 30 Min. nach Gabe von Tetrabenazin wurde die Ptosis entsprechend der folgenden Skala beurteilt:

| Ptosis | Score |
|---|---|
| Augen geschlossen | 4 |
| Augen $\frac{3}{4}$ geschlossen | 3 |
| Augen $\frac{1}{2}$ geschlossen | 2 |
| Augen $\frac{1}{4}$ geschlossen | 1 |
| Augen offen | 0 |

Die Ptosis-Scores wurden kumuliert und das Ergebnis der Gruppen auf den maximal erreichbaren Score (6 x 4 = 100 %) bezogen. Mittels linearer Regression wurde die $ED_{50}$ (mg/kg) ermittelt als die Dosis, bei der der Grad der Ptosis 50 % des bei der Kontrollgruppe ermittelten Wertes beträgt.

2. Potenzierung der Yohimbin-Toxizität an Mäusen

Die Toxizität von Yohimbin, einem Blocker präsynaptischer alpha-Rezeptoren, wird durch Antidepressiva potenziert (Literatur R.M. Quinton, Brit. J. Pharmac. 21 , 55-56 (1963)). Männliche Mäuse, NMRI-Stamm, 20-22 g, wurden in Versuchsgruppen zu n = 10 zugeordnet. Das Prüfpräparat wurde in 1 %igem wäßrigen

Tylose®-Schleim suspendiert und im Volumen 10 ml/kg oral gegeben. Kontrollen erhielten nur das Vehikel.

Nach einer Vorbehandlungszeit von 1 Stunde erhielten alle Tiere eine allein nicht toxische Dosis von Yohimbin-Hydrochlorid, 20 mg/kg subkutan. Die verstorbenen Tiere wurden 18 Stunden nach dieser Behandlung ermittelt. (Berechnung der $LD_{50}$ mit Probit-Analyse).

3. Prüfung der Auslösung von Stereotypien an Ratten

Die Versuche wurden an Wistar-Ratten, männlich, 130-140 g durchgeführt. Die Stereotypien wurden in den ersten beiden Stunden nach Substanzgabe in 15-Minuten-Intervallen beurteilt, danach bis zur 6. Stunde in halbstündigen Intervallen. Die folgende Skala wurde benutzt.

| Symptome | Punktzahl |
|---|---|
| Verhalten wie Kontrollgruppe | 0 |
| Gesteigertes Schnüffeln | 1 |
| Schnüffeln und Aufrichten | 2 |
| Verstärktes Laufen, vereinzelt Kopf und Vorderpfoten geschüttelt | 3 |
| gelegentliches Lecken, Beißen, Kauen | 4 |
| fortgesetzt wie 4 | 5 |

Die Punktzahlen wurden errechnet alß Gesamtzahl pro 6 Stunden, dividiert durch die Zahl der Beobachtungen.

Der $ED_{50}$-Wert ist die Dosis, bei der die Hälfte der maximal möglichen Punktzahl (5 Punkte multipliziert mit der Zahl der Beobachtungen) erreicht wird.

4. Wiederaufnahmehemmung von Noradrenalin in Synaptosomen Methode

Synaptosomen aus Rattenhirn werden nach der Methode von Whittaker (Handbook of Neurochemistry 2, 327-364, Editor A. Lajtha; London and New York, 1969) isoliert und die Monoamin-Aufnahme nach der Methode von Schacht und Heptner gemessen (Biochemical Pharmac. 23, 3413-3422). Die $^{14}C$ Noradrenalin-Aufnahme wurde in einem Krebs-Henseleit-Bikarbonatpuffer pH 7,4, der 11 Millimol Glucose enthielt, gemessen. 2,5 ml der Synaptosomen-Suspension wurden mit markiertem Noradrenalin bei 37 °C inkubiert in Gegenwart von oder ohne Testsubstanz. Die Inkubationszeit betrug 4 Minuten. Die weitere Aufnahme wurde dann durch Abkühlen mit Eis gestoppt. Um nichtspezifische Adsorptionen auszuschließen, wurden Kontrollproben bei 0 °C unter sonst gleichen Bedingungen inkubiert.

Die aufgenommenen Noradrenalinmengen wurden mit Hilfe der Membranfiltrationstechnik mit einem Millipore Sampling Manifold mit Cellulosenitratfiltern von 25 mm Durchmesser und 0,6 Mikrometer Porengröße gemessen. Die Synaptosomen wurden unter vermindertem Druck gesammelt und die Radioaktivität in einem Packard-Tricarb-Scintillationszähler bestimmt. Die Menge an angesammeltem Noradrenalin wurde angegeben als Prozent Radioaktivität, die zur Inkubationsmischung zugesetzt wurde.

Die $IC_{50}$-Werte (inhibitory concentration) der nachstehenden Tabellen geben die Konzentrationen der Testsubstanzen an, welche die Aufnahme von $^{14}C$-Noradrenalin zu 50 % hemmen.

| Ergebnisse der pharmakologischen Prüfung | | |
|---|---|---|
| (+)-(2-Hydroxy-2-adamantyl)-1-imidazolyl-3-tolyl-methan-(2S,3S)-hydrogentartrat (nach Beispiel 1) | | |
| | $ED_{50}$; mg/kg p.o. (Vertrauensgrenzen) | $ED_{50}$; mg/kg umgerechnet (F:O,68) auf äquimolare Menge Base (nach Beispiel 2) |
| Methode 1, Maus | 1,3 (1,0 - 1,6)<br>1,2 (0,3 - 3,5)<br>1,75 (0,4 - 6,9)<br>1,9 (1,0 - 3,5)<br>1,4 (0,7 - 2,7) | 0,9 (0,7 - 1,1)<br>0,8 (0,2 - 2,4)<br>1,2 (0,3 - 4,7)<br>1,3 (0,7 - 2,4)<br>1,0 (0,5 - 1,8) |
| Methode 2, Maus | $LD_{50}$, mg/kg p.o.<br>2,9 (0,2 - 41,3) | 2,0 (0,1 - 28,1) |
| Methode 3, Ratte | keine Stereotypien bis 100 mg/kg p.o. | |
| Methode 4, $IC_{50} = 7 \cdot 10^{-8}$ M | | |

Ergebnisse der pharmakologischen Prüfung von stark wirksamen Verbindungen der gleichen Stoffklasse (wie die erfindungsgemäße Verbindung) als Vergleich:
(Soweit nicht anders erwähnt, handelt es sich bei den hier aufgeführten Verbindungen um Racemate)

1. 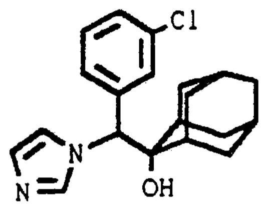

Methode 1 (Vertrauensgrenzen)
Maus $ED_{50}$ 0,8 mg/kg p.o.
$ED_{50}$ 1,9 (1,4-2,7) mg/kg p.o.
Methode 2, Maus $LD_{50}$ 4,4 mg/kg p.o.
Methode 3, Ratte: keine Stereotypien bis 300 mg/kg p.o.
Methode 4: $IC_{50} = 4 \cdot 10^{-8}$ M
$IC_{50} = 15 \cdot 10^{-8}$ M

2. 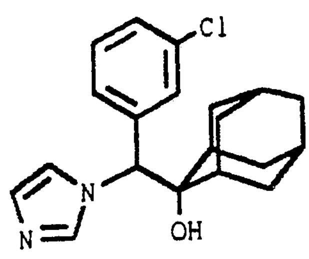 x 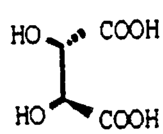

(+)-Enantiomer

Methode 1 Maus $ED_{50}$ 2,9 (0,8-9,9) mg/kg p.o.
$ED_{50}$ 2,3 (0,9-5,8) mg/kg p.o.
Methode 2, Maus $LD_{50}$ 4,6 mg/kg p.o. 8,5 (0,5-130) mg/kg p.o.
Methode 3, Ratte: keine Stereotypien bis 30 mg/kg p.o.
Methode 4: $IC_{50} = 12 \cdot 10^{-8}$ M

*) Im: 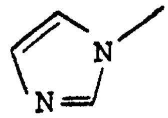

3. 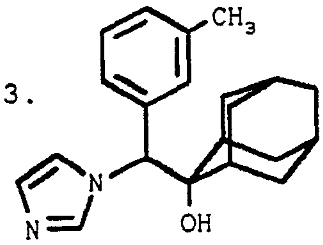

Methode 1, Maus $ED_{50}$ 1,5 mg/kg p.o. 1,7 (0,5-5,9) mg/kg p.o.
Methode 2, Maus $LD_{50}$ 7,1 mg/kg p.o.
Methode 3, Ratte: keine Stereotypien bis 30 mg/kg p.o.
Methode 4: $IC_{50} = 12 \cdot 10^{-8}$ M

4. 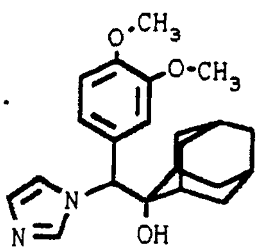

Methode 1, Maus $ED_{50}$ 3,0 mg/kg p.o.
Methode 2, Maus $LD_{50}$ 3,0 mg/kg p.o.

5. 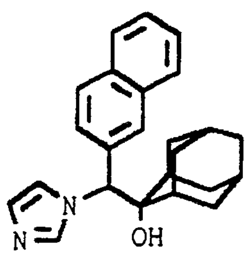

Methode 1, Maus $ED_{50}$ 3,0 mg/kg p.o.
Methode 2, Maus $LD_{50}$ 3,2 mg/kg p.o.

6. 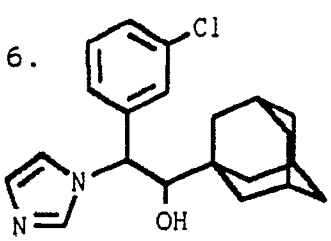

Methode 1, Maus $ED_{50}$ 3,7 mg/kg p.o.
Methode 2, Maus $LD_{50}$ 8,5 mg/kg p.o.
Methode 3, Ratte: keine Stereotypien bis 30 mg/kg p.o.

7.

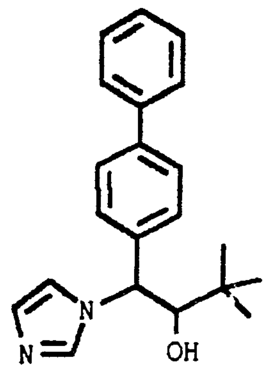

Methode 1, Maus $ED_{50}$ 3,0 mg/kg p.o.
Methode 2, Maus $LD_{50}$ 5,3 mg/kg p.o.
Methode 3, Ratte: keine Stereotypien bis 30 mg/kg p.o.
Methode 4, $IC_{50} > 1 \cdot 10^{-6}$ M

8.

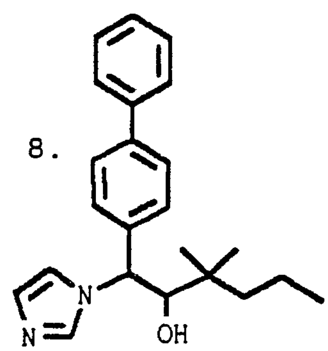

Methode 1, Maus $ED_{50}$ 1,2 (0,3-5,4) mg/kg p.o.
2,1 (0,7-5,8) mg/kg p.o.
5,7 (2,0-16,2) mg/kg p.o.
Methode 2, Maus $LD_{50}$ 24,5 mg/kg p.o.
Methode 3, Ratte: keine Stereotypien bis mg/kg p.o.

9.

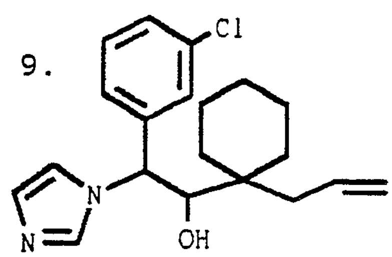

Methode 1, Maus $ED_{50}$ 3,9 (2,1-7,3) mg/kg p.o.
6,1 (0,5-72,9) mg/kg p.o.

## Ansprüche

1. Das rechtsdrehende, das (+)-Enantiomere des (2-Hydroxy-2-adamantyl)-1-imidazolyl-3-tolyl-methans der Formel I

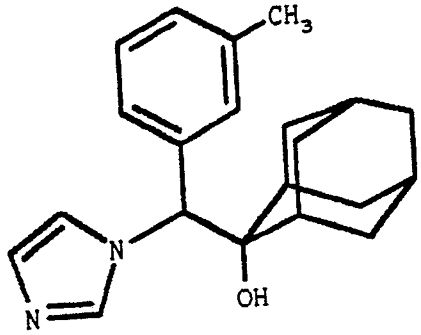

I

sowie die physiologisch verträglichen Säureadditionssalze.

2. Das (2S,3S)-Hydrogentartrat der Verbindung der Formel I nach Anspruch 1.

3. Das Hydrochlorid der Verbindung der Formel I nach Anspruch 1.

4. Verfahren zur Herstellung des rechtsdrehenden Enantiomeren des (2-Hydroxy-2-adamantyl)-1-imidazolyl-3-tolyl-methans der Formel I, sowie dessen physiologisch verträglichen Säureadditionssalze, dadurch gekennzeichnet, daß man das Racemat der Verbindung I mit einem Äquivalent (2S,3S)-(-)-Weinsäure in einem geeigneten Lösungsmittel oder Lösungsmittelgemisch in das Hydrogentartrat überführt und das gebildete diastereomere Hydrogentartrat aus einem geeigneten Lösungsmittel oder Lösungsmittelgemisch fraktioniert kristallisieren läßt und anschließend das auskristallisierte rechtsdrehende (+)-(2-Hydroxy-2-adamantyl)-1-imidazolyl-3-tolyl-methan-(2S,3S)-hydrogentartrat ein- oder mehrmals aus einem geeigneten Lösungsmittel oder Lösungsmittelgemisch umkristallisiert bis die spezifische Drehung konstant bleibt, und gegebenenfalls das so erhaltene enantiomeren-reine Hydrogentartrat in die freie Base des (+)-(2-Hydroxy-2-adamantyl)-1-imidazolyl-3-tolyl-methans überführt und aus dieser gegebenenfalls ein anderes physiologisch verträgliches Säureadditionssalz herstellt.

5. Verwendung einer oder mehrerer Verbindungen der Formel I nach den Ansprüchen 1, 2 oder 3 als Antidepressivum.

6. Verwendung einer oder mehrerer Verbindungen der Formel I nach den Ansprüchen 1, 2 oder 3 zur Herstellung eines antidepressiv wirkenden Arzneimittels.

7. Arzneimittel mit antidepressiver Wirkung, gekennzeichnet durch einen wirksamen Gehalt an einer oder mehreren Verbindungen der Formel I nach den Ansprüchen 1, 2 oder 3.

8. Verfahren zum Behandeln von depressiven Zuständen, dadurch gekennzeichnet, daß man eine wirksame Menge einer oder mehrerer Verbindungen der Formel I nach den Ansprüchen 1, 2 oder 3, gegebenenfalls zusammen mit pharmazeutisch geeigneten Trägerstoffen appliziert.

Patentansprüche für folgenden Vertragsstaat: ES

1. Verfahren zur Herstellung des rechtsdrehenden Enantiomeren des (2-Hydroxy-2-adamantyl)-1-imidazolyl-3-tolyl-methans der Formel I,

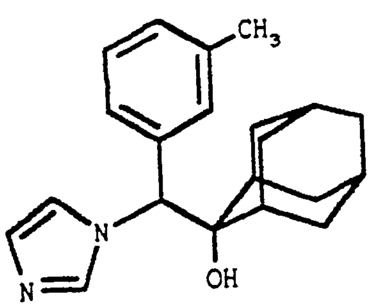

I

sowie die physiologisch verträglichen Säureadditionssalze, dadurch gekennzeichnet, daß man das Racemat der Verbindung I mit einem Äquivalent (2S,3S)-(-)-Weinsäure in einem geeigneten Lösungsmittel oder Lösungsmittelgemisch in das Hydrogentartrat überführt und das gebildete diastereomere Hydrogentartrat aus einem geeigneten Lösungsmittel oder Lösungsmittelgemisch fraktioniert kristallisieren läßt und anschließend das auskristallisierte rechtsdrehende (+)-(2-Hydroxy-2-adamantyl)-1-imidazolyl-3-tolyl-methan-(2S,3S)-hydrogentartrat ein- oder mehrmals aus einem geeigneten Lösungsmittel oder Lösungsmittelgemisch umkristallisiert bis die spezifische Drehung konstant bleibt, und gegebenenfalls das so erhaltene enantiomeren-reine Hydrogentartrat in die freie Base des (+)-(2- Hydroxy-2-adamantyl)-1-imidazolyl-3-tolylmethans überführt und aus dieser gegebenenfalls ein anderes physiologisch verträgliches Säureadditionssalz herstellt.

2. Verfahren nach Anspruch 1, dadurch gekennzeichnet, daß es sich um das (2S,3S)-Hydrogentartrat der Verbindung der Formel I handelt.

3. Verfahren nach Anspruch 1, dadurch gekennzeichnet, daß es sich um das Hydrochlorid der Verbindung der Formel I handelt.

4. Verwendung einer oder mehrerer Verbindungen der Formel I nach den Ansprüchen 1, 2 oder 3 als Antidepressivum.

5. Verwendung einer oder mehrerer Verbindungen der Formel I nach den Ansprüchen 1, 2 oder 3 zur Herstellung eines antidepressiv wirkenden Arzneimittels.

6. Arzneimittel mit antidepressiver Wirkung, gekennzeichnet durch einen wirksamen Gehalt an einer oder mehreren Verbindungen der Formel I nach den Ansprüchen 1, 2 oder 3.

7. Verfahren zum Behandeln von depressiven Zuständen, dadurch gekennzeichnet, daß man eine wirksame Menge einer oder mehrerer Verbindungen der Formel I nach den Ansprüchen 1, 2 oder 3, gegebenenfalls zusammen mit pharmazeutisch geeigneten Trägerstoffen appliziert.